(19) **Europäisches Patentamt**
**European Patent Office**
**Office européen des brevets**

(11) **EP 3 370 592 B1**

(12) **EUROPEAN PATENT SPECIFICATION**

(45) Date of publication and mention
of the grant of the patent:
**11.01.2023 Bulletin 2023/02**

(21) Application number: **16862884.0**

(22) Date of filing: **02.11.2016**

(51) International Patent Classification (IPC):
*D21H 17/25* (2006.01)     *D21H 17/46* (2006.01)
*D21H 21/22* (2006.01)     *D21H 27/40* (2006.01)
*A61L 15/28* (2006.01)     *D21H 27/00* (2006.01)
*D21H 21/56* (2006.01)     *A61L 15/42* (2006.01)
*A61F 15/00* (2006.01)     *A61F 13/53* (2006.01)
*A61F 13/534* (2006.01)     *A61F 13/537* (2006.01)

(52) Cooperative Patent Classification (CPC):
**A61F 15/001; A61F 13/537; A61F 13/5376;**
**A61L 15/28; A61L 15/425; D21H 13/00;**
**D21H 13/14; D21H 13/24; D21H 13/26;**
**D21H 13/34; D21H 21/20; D21H 21/22;**
**D21H 21/56; D21H 27/002; D21H 27/40;**    (Cont.)

(86) International application number:
**PCT/US2016/060128**

(87) International publication number:
**WO 2017/079310 (11.05.2017 Gazette 2017/19)**

(54) **FOAMED COMPOSITE WEB WITH LOW WET COLLAPSE**

GESCHÄUMTE VERBUNDBAHN MIT ZUSAMMENFALLEN BEI GERINGER FEUCHTIGKEIT

TOILE COMPOSITE EXPANSÉE AYANT UN FAIBLE AFFAISSEMENT À L'HUMIDITÉ

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **03.11.2015 US 201562250227 P**

(43) Date of publication of application:
**12.09.2018 Bulletin 2018/37**

(60) Divisional application:
**22205540.2**

(73) Proprietor: **Kimberly-Clark Worldwide, Inc.**
**Neenah, Wisconsin 54956 (US)**

(72) Inventors:
 • **QIN, Jian**
 **Appleton, Wisconsin 54915 (US)**
 • **CALEWARTS, Deborah J.**
 **Winneconne, Wisconsin 54986 (US)**
 • **WALDROUP, Donald E.**
 **Roswell, Georgia 30075 (US)**

(74) Representative: **Dehns**
**St. Bride's House**
**10 Salisbury Square**
**London EC4Y 8JD (GB)**

(56) References cited:
**WO-A1-01/12902**     **WO-A1-98/24621**
**WO-A2-02/055788**     **US-A- 5 571 618**
**US-A1- 2003 018 311**     **US-A1- 2008 121 461**
**US-A1- 2014 001 196**     **US-B1- 6 346 557**
**US-B1- 6 550 634**

(52) Cooperative Patent Classification (CPC): (Cont.)
A61F 2013/530007; A61F 2013/530649;
A61F 2013/53445

## Description

### Background

Wood pulp fiber is a natural fiber widely used in personal care products and other home uses due to its having advantageous properties such as being highly absorbent, relatively soft and biodegradable. Further, wood pulp fibers are a readily available renewable resource that is relatively inexpensive. As specific examples, wood pulp fibers are commonly used in absorbent cores for diapers and also in wipes. However, wood pulp fibers are not wet stable and the bending stiffness of wood pulp fibers significantly degrades when wet. This property is problematic for many articles. In this regard, many applications and/or products employing wood pulp fibers require maintenance of the liquid handling properties and/or bulk after initial wetting. For example, absorbent personal care articles such as diapers are often worn for extended periods of time and must be capable of effectively handling and absorbing multiple wettings or insults. Similarly, in various wiping applications, it is desired that the liquid handling properties and bulk not be lost upon initial wetting as the wipe is often used to clean certain surfaces only after an aqueous cleaning composition has been applied thereto. Loss of the bulky, open structure can reduce the cleaning efficacy and/or durability of wipes that include substantial amounts of wood pulp.

The wet stability of webs including wood pulp have been attempted to be improved by the inclusion of various binders and adhesives. As an example, structures made by an air-laying process act to inter-mix both cellulosic fibers and synthetic fibers to form a nonwoven web. With respect to nonwoven webs formed by air laying process, such webs tend to have high bulk as the air-laying processes typically achieves an open and non-oriented fiber structure. However, when such air-laid webs have significant levels of wood pulp fibers they still often suffer from low wet integrity, often collapsing (i.e. losing bulk) upon wetting. While the wet integrity of air-laid webs can be improved by reducing the amount of wood pulp fibers and adding significant levels of binder fibers or adhesive, the change in the nonwoven web composition often causes the nonwoven web to lose desired attributes such as absorbency, softness, and/or flexibility. WO 98/24621 discloses a unitary stratified composite and methods for forming said unitary stratified composite.

Thus, there exists a need for absorbent composite materials that can achieve desired combinations of bulk, absorbency and softness and that retain wet stability rendering them better suited for use in personal care products and other applications.

### Summary of Invention

The present invention provides an absorbent composite foam comprising: (i) between 5 to 40% by wt. water resistant fibers; (ii) between 30 to 45% by wt. cellulosic fibers; (iii) between 5 to 35% by wt. binder; (iv) a foaming surfactant. In the absorbent composite materials of the present invention, inter-fiber bonding is achieved in at least two respects, the binder which forms bonds between adjacent fibers at fiber contact points and also by hydrogen bonding formed by the as between adjacent cellulosic fibers. The absorbent composite foam can therefore provide an ultra-low density, being between 0.04 g/cc and 0.005 g/cc, and yet still also provide a Wet Caliper Retention greater than 40%. In certain embodiments, the absorbent composite foam can have a density between about 0.02 g/cc and about 0.005 g/cc. Further, the absorbent composite foam may comprise between about 0.05 and about 5% by weight foaming surfactant. In further embodiments, the absorbent composite foam may comprise a majority of wood pulp fibers and have a wet retention value in excess of about 60%.

In certain embodiments, a multi-layer laminate is also provided having a first layer comprising the absorbent composite foam described herein and a second layer comprising a nonwoven web selected from the group of wet-laid webs, air-laid webs, and hydroentangled webs. In a particular embodiment, the nonwoven web of the second layer can comprise a paper tissue web having a density of between about 0.5 g/cc and about 0.04 g/cc and that comprises at least about 95% cellulosic fibers. In still other embodiments, the absorbent foam composite and multi-layer laminates described herein may be employed as dispensable wipers including, for example, within a dispensable stack of wet wipes. In still further embodiments, the absorbent foam composite and multi-layer laminates described herein may be employed as part of an absorbent core or liquid distribution layer of an absorbent personal care product such as, for example, within a diaper or feminine care article.

### Figures

Figure 1 is a perspective view of a dispenser housing a stack of absorbent composite sheets of the present invention.

Figure 2 is a top, plan view of an absorbent personal care article including an absorbent composite material of the present invention.

## Description

The low basis weight, wet stable absorbent composite materials of the present invention are made by forming a highly-expanded, highly porous foam from a foam precursor or slurry comprising (i) a foaming fluid; (ii) fluid resistant fibers, (iii) cellulosic fibers; (iv) binder; (v) foaming surfactant and, optionally one or more (vi) foam stabilizers; and/or (vii) additional additives.

Foam Precursor / Slurry

Foaming Fluid

Any one or more known foaming fluids compatible with the other components may be used in the practice of the present invention. Suitable carriers include, but are not limited to, water. The foaming fluid desirably comprises at least about 85% of the slurry. In certain embodiments, the foaming fluid can comprise between about 90% and about 99.9% % of the slurry. In certain other embodiments, the foaming fluid can comprise between about 93% and 99.5% of the slurry or even between about 95% and about 99.0% of the slurry.

Fluid Resistant Fibers

Fluid resistant fibers include those that are resistant to the forming fluid, namely those that are non-absorbent and whose bending stiffness is substantially unimpacted by the presence of forming fluid. As noted above, typically the forming fluid will comprise water. By way of non-limiting example, water-resistant fibers include fibers such as polymeric fibers comprising polyolefin, polyester, polyamide, polylactic acid, or other fiber forming polymers. Polyolefin fibers, such as polyethylene and polypropylene, are particularly well suited for use in the present invention. In addition, highly crosslinked cellulosic fibers having no-significant absorbent properties can also be used herein. In this regard, due to its very low levels of absorbency to water, water resistant fibers do not experience a significant change in bending stiffness upon contacting an aqueous fluid and therefore are capable of maintain an open composite structure upon wetting. Water resistant fibers desirably have a water retention value (WRV) less than about 1 and still more desirably between about 0 and about 0.5. In certain aspects, it is desirable that the fibers be non-absorbent fibers.
The synthetic fibers can have fiber length greater than about 0.2 mm including, for example, having an average fiber size between about 0.5 mm and about 50 mm or between about 0.75 and about 30 mm or even between about 1 mm and about 25 mm.

Cellulosic Fibers

A wide variety of cellulosic fibers are believed suitable for use herein. The ultra-low density cellulosic webs of the present invention can, in one aspect, comprise conventional papermaking fibers such as wood pulp fibers formed by a variety of pulping processes, such as kraft pulp, sulfite pulp, bleached chemithermomechanical pulp (BCTMP), chemithermomechanical pulp (CTMP), pressure/pressure thermomechanical pulp (PTMP), thermomechanical pulp (TMP), and thermomechanical chemical pulp (TMCP). By way of example only, fibers and methods of making wood pulp fibers are disclosed in US4793898 to Laamanen et al.; US4594130 to Chang et al.; US3585104 to Kleinhart; US5595628 to Gordon et al.; US5522967 to Shet. Further, the fibers may be any high-average fiber length wood pulp, low-average fiber length wood pulp, or mixtures of the same. Examples of suitable high-average length pulp fibers include softwood fibers, such as, but not limited to, northern softwood, southern softwood, redwood, red cedar, hemlock, pine (e.g., southern pines), spruce (e.g., black spruce), and the like. Examples of suitable low-average length pulp fibers include hardwood fibers, such as, but not limited to, eucalyptus, maple, birch, aspen, and the like.
Moreover, if desired, secondary fibers obtained from recycled materials may be used, such as fiber pulp from sources such as, for example, newsprint, reclaimed paperboard, and office waste. In a particularly preferred embodiment refined fibers are utilized in the tissue web such that the total amount of virgin and/or high average fiber length wood fibers, such as softwood fibers, may be reduced.
Regardless of the origin of the wood pulp fiber, the wood pulp fibers preferably have an average fiber length greater than about 0.2 mm and less than about 3 mm, such as from about 0.35 mm and about 2.5 mm, or between about 0.5 mm to about 2 mm or even between about 0.7 mm and about 1.5 mm.
In addition, other cellulosic fibers believed suitable for use in making the cellulosic webs of the present invention include nonwoody fibers, such as cotton, abaca, bambo, kenaf, sabai grass, flax, esparto grass, straw, jute hemp, bagasse, milkweed floss fibers, and pineapple leaf fibers. Still further, other cellulosic fibers for making the tissue webs include synthetic cellulose fiber types including rayon in all its varieties and other fibers derived from viscose or chemically-modified cellulose such as, for example, those available under the trade names LYOCELL and TENCEL. The non-woody and synthetic cellulosic fibers can have fiber length greater than about 0.2 mm including, for example, having an average fiber size between about 0.5 mm and about 50 mm or between about 0.75 and about 30 mm or even between about 1 mm and about 25 mm. Generally speaking, when fibers of relatively larger average length are being used, it will often be advantageous to utilize relatively higher amounts of foaming surfactant in order to help achieve a foam with the required stability.

The solids content, including the water-resistant fibers, cellulosic fibers and any other fibers or particulates contained herein, desirably comprise no more than about 15% of the slurry. In certain embodiments the cellulosic fibers can comprise between about 0.1% and about 15% of the slurry or between about 0.2 and about 10% of the slurry or even between about 0.5% and about 8% of the slurry.

Binder

Binder materials suitable for use in the present invention include, but are not limited to, thermoplastic binder fibers and water-compatible adhesives such as, for example, latexes. Importantly, the binder will comprise one that is water insoluble on the dried cellulosic web. In certain embodiments, latexes used in the present invention can be cationic or anionic to facilitate application to and adherence to the cellulosic fiber. For instance, latexes believed suitable for use with the present invention include, but are not limited to, anionic styrene-butadiene copolymers, polyvinyl acetate homopolymers, vinyl-acetate ethylene copolymers, vinyl-acetate acrylic copolymers, ethylene-vinyl chloride copolymers, ethylene-vinyl chloride-vinyl acetate terpolymers, acrylic polyvinyl chloride polymers, acrylic polymers, nitrile polymers, as well as other suitable anionic latex polymers known in the art. Examples of such latexes are described in US4785030 to Hager, US6462159 to Hamada, and US6752905 to Chuang et al. Examples of suitable thermoplastic binder fibers include, but are not limited to, monocomponent and multi-component fibers having at least one relatively low melting thermoplastic polymer such as polyethylene. In certain embodiments, polyethylene/polypropylene sheath/core staple fibers can be used. Binder fibers may have lengths in line with those described herein above in relation to the synthetic cellulosic fibers.

Binders in liquid form, such as latex emulsions, can comprise between about 0% and about 10 % of the slurry. In certain embodiments the non-fibrous binder can comprise between about 0.1% and 10% of the slurry or even between about 0.2% and about 5% or even between about 0.5% and about 2% of the slurry. Binder fibers, when used, may be added proportionally to the other components to achieve the desired fiber ratios and structure while maintaining the total solids content of the slurry below the amounts stated above.

Foaming Surfactant

The ultra-low density paper tissue webs of the present invention are made utilizing one of more surfactants capable of forming a stable, high-expansion foam. The fibers and surfactant, together with the foaming liquid and any additional components, need to form a stable dispersion capable of substantially retaining a high degree of porosity for longer than the drying process. In this regard, the surfactant is selected so as to provide a foam slurry having a stability of at least 15 minutes, and still more desirably at least 30 minutes. The foaming surfactant used in the slurry can be selected from one or more known in the art that are capable of providing the desired degree of foam stability. In this regard, the foaming surfactant can be selected from anionic, cationic, nonionic and amphoteric surfactants provided they, alone or in combination with other components, provide the necessary foam stability. As will be appreciated, more than one surfactant can be used, including both different types of surfactants and more than one surfactant of the same type.

Anionic surfactants believed suitable for use with the present invention include, without limitation, anionic sulfate surfactants, alkyl ether sulfonates, alkylaryl sulfonates, or mixtures or combinations thereof. Examples of alkylaryl sulfonates include, without limitation, alkyl benzene sulfonic acids and their salts, dialkylbenzene disulfonic acids and their salts, dialkylbenzene sulfonic acids and their salts, alkylphenol sulfonic acids/condensed alkylphenol sulfonic acids and their salts, or mixture or combinations thereof. Examples of additional anionic surfactants believed suitable for use in the present invention include alkali metal sulforicinates, sulfonated glyceryl esters of fatty acids such as sulfonated monoglycerides of coconut oil acids, salts of sulfonated monovalent alcohol esters such as sodium oleylisethianate, metal soaps of fatty acids, amides of amino sulfonic acids such as the sodium salt of oleyl methyl tauride, sulfonated products of fatty acids nitriles such as palmitonitrile sulfonate, alkali metal alkyl sulfates such as sodium lauryl sulfate, ammonium lauryl sulfate or triethanolamine lauryl sulfate, ether sulfates having alkyl groups of 8 or more carbon atoms such as sodium lauryl ether sulfate, ammonium lauryl ether sulfate, sodium alkyl aryl ether sulfates, and ammonium alkyl aryl ether sulfates, sulphuric esters of polyoxyethylene alkyl ether, sodium salts, potassium salts, and amine salts of alkylnapthylsulfonic acid. Certain phosphate surfactants including phosphate esters such as sodium lauryl phosphate esters or those available from the Dow Chemical Company under the tradename TRITON are also believed suitable for use herewith. A particularly desired anionic surfactant is sodium dodecyl sulfate (SDS).

Cationic surfactants are also believed suitable for use with the present invention. Foaming cationic surfactants include, without limitation, monocarbyl ammonium salts, dicarbyl ammonium salts, tricarbyl ammonium salts, monocarbyl phosphonium salts, dicarbyl phosphonium salts, tricarbyl phosphonium salts, carbylcarboxy salts, quaternary ammonium salts, imidazolines, ethoxylated amines, and quaternary phospholipids. Examples of additional cationic surfactants include various fatty acid amines and amides and their derivatives, and the salts of the fatty acid amines and amides. Examples of aliphatic fatty acid amines include dodecylamine acetate, octadecylamine acetate, and acetates of the amines of tallow fatty acids, homologues of aromatic amines having fatty acids such as dodecylanalin, fatty amides derived from aliphatic diamines such as undecylimidazoline, fatty amides derived from aliphatic diamines such as undecylimidazoline, fatty amides derived from disubstituted amines such as oleylaminodiethylamine, derivatives of ethylene diamine, quaternary ammonium compounds and their salts which are exemplified by tallow trimethyl ammonium chloride, dioctadecyldimethyl ammonium chloride, didodecyldimethyl ammonium chloride, dihexadecyl ammonium chloride, alkyltrimethylammonium hydroxides, dioctadecyldimethylammonium hydroxide, tallow trimethylammonium hydroxide, trimethylammonium hydroxide, methylpolyoxyethylene cocoammonium chloride, and dipalmityl hydroxyethylammonium methosulfate, amide derivatives of amino alcohols such as beta-hydroxylethylstearylamide, and amine salts of long chain fatty acids. Further examples of cationic surfactants believed suitable for use with the present invention include benzalkonium chloride, benzethonium chloride, cetrimonium bromide, distearyldimethylammonium chloride, and tetramethylammonium hydroxide.

Nonionic surfactants believed suitable for use in the present invention include, without limitation, condensates of ethylene oxide with a long chain fatty alcohol or fatty acid, condensates of ethylene oxide with an amine or an amide, condensation products of ethylene and propylene oxides, fatty acid alkylol amide and fatty amine oxides. Various additional examples of non-ionic surfactants include stearyl alcohol, sorbitan monostearate, octyl glucoside, octaethylene glycol monododecyl ether, lauryl glucoside, cetyl alcohol, cocamide MEA, monolaurin, polyoxyalkylene alkyl ethers such as polyethylene glycol long chain (12-14C) alkyl ether, polyoxyalkylene sorbitan ethers, polyoxyalkylene alkoxylate esters, polyoxyalkylene alkylphenol ethers, ethylene glycol propylene glycol copolymers, polyvinyl alcohol, alkylpolysaccharides, polyethylene glycol sorbitan monooleate, and octylphenol ethylene oxide.

The foaming surfactant can be used in varying amounts as necessary to achieve the desired foam stability and air-content. In certain embodiments, the foaming surfactant can comprise between about 0.01 % and about 5% of the slurry. In certain embodiments the foaming surfactant can comprise between about 0.05% and about 3% of the slurry or even between about 0.1 % and about 2% of the slurry.

Foam Stabilizers

The foam pre-cursor or slurry may optionally also include one or more foam stabilizers known in the art and that are compatible with the components of the slurry and further do not interfere with the hydrogen bonding as between the cellulosic fibers. Foam stabilizing agents believed suitable for use in the present invention, without limitation, one or more zwitterionic compounds, amine oxides, alkylated polyalkylene oxides, or mixture or combinations thereof. Specific examples of foam stabilizers includes, without limitation, cocoamine oxide, isononyldimethylamine oxide, and n-dodecyldimethylamine oxide.

The foam stabilizer can comprise between about 0.01% and about 2 % of the slurry. In certain embodiments the foam stabilizer can comprise between about 0.05% and 1% of the slurry or even between about 0.1 and about 0.5% of the slurry.

Additional Additives

The foam-precursor may, optionally, include one or more absorbent materials or particles such as those commonly known as superabsorbent materials (SAM). SAM is commonly provided in a particulate form and, in certain aspects, can comprise polymers of unsaturated carboxylic acids or derivatives thereof. These polymers are often rendered water insoluble, but water swellable, by crosslinking the polymer with a di- or polyfunctional internal crosslinking agent. These internally crosslinked polymers are at least partially neutralized and commonly contain pendant anionic carboxyl groups on the polymer backbone that enable the polymer to absorb aqueous fluids, such as body fluids. Typically, the SAM particles are subjected to a post-treatment to crosslink the pendant anionic carboxyl groups on the surface of the particle. SAMs are manufactured by known polymerization techniques, desirably by polymerization in aqueous solution by gel polymerization. The products of this polymerization process are aqueous polymer gels, i.e., SAM hydrogels that are reduced in size to small particles by mechanical forces, then dried using drying procedures and apparatus known in the art. The drying process is followed by pulverization of the resulting SAM particles to the desired particle size. Examples of superabsorbent materials include, but are not limited to, those described in US7396584 Azad et al.,

US7935860 Dodge et al., US2005/5245393 to Azad et al., US2014/09606 to Bergam et al., and WO2008/027488 to Chang et al. In addition, in order to aid processing, the SAM may be treated in order to render the material temporarily non-absorbing during the formation of the slurry and formation of the highly-expanded foam. For example, in one aspect, the SAM may be treated with a water-soluble protective coating having a rate of dissolution selected such that the SAM is not substantially exposed to the aqueous carrier until the highly-expanded foam has been formed and drying operations initiated. Alternatively, in order to prevent or limit premature expansion during processing, the SAM may be introduced into the process at low temperatures.

One or more wet strength additives may, optionally, be added to the foam precursor or slurry in order to help improve the relative strength of the ultra-low density composite cellulosic material. Such strength additives suitable for use with paper making fibers and the manufacture of paper tissue are known in the art. Temporary wet strength additives may be cationic, nonionic or anionic. Examples of such temporary wet strength additives include PAREZ™ 631 NC and PAREZ(R) 725 temporary wet strength resins that are cationic glyoxylated polyacrylamides available from Cytec Industries, located at West Paterson, N.J. These and similar resins are described in US3556932 to Coscia et al. and US3556933 to Williams et al. Additional examples of temporary wet strength additives include dialdehyde starches and other aldehyde containing polymers such as those described in US6224714 to Schroeder et al.; US6274667 to Shannon et al.; US6287418 to Schroeder et al.; and US6365667 to Shannon et al.

Permanent wet strength agents comprising cationic oligomeric or polymeric resins may also be used in the present invention. Polyamide-polyamine-epichlorohydrin type resins such as KYMENE 557H sold by Hercules, Inc. located at Wilmington, Del. are the most widely used permanent wet-strength agents and are suitable for use in the present invention. Such materials have been described in the following US3700623 to Keim; US3772076 to Keim; US3855158 to Petrovich et al.; US3899388to Petrovich et al.; US4129528 to Petrovich et al.; US4147586 to Petrovich et al.; and US4222921 to van Eenam. Other cationic resins include polyethylenimine resins and aminoplast resins obtained by reaction of formaldehyde with melamine or urea. Permanent and temporary wet strength resins may be used together in the manufacture of composite cellulosic products of the present invention. Further, dry strength resins may also optionally be applied to the composite cellulosic webs of the present invention. Such materials may include, but are not limited to, modified starches and other polysaccharides such as cationic, amphoteric, and anionic starches and guar and locust bean gums, modified polyacrylamides, carboxymethylcellulose, sugars, polyvinyl alcohol, chitosan, and the like.

Such wet and dry strength additives can comprise between about 0.01 and about 5% of the slurry. In certain embodiments the strength additives can comprise between about 0.05% and about 2% of the slurry or even between about 0.1 % and about 1 % of the slurry.

Still other additional components may be added to the slurry so long as they do not significantly interfere with the formation of the highly-expanded stable foam, the hydrogen bonding as between the cellulosic fibers or other desired properties of the web. As examples, additional additives may include one or more pigments, opacifying agents, anti-microbial agents, pH modifiers, skin benefit agents, and fragrances, as desired to impart or improve one or more physical or aesthetic attributes. In certain embodiments the composite cellulosic webs may include skin benefit agents such as, for example, antioxidents, astringents, conditioners, emollients, deodorants, external analgesics, film formers, humectants, hydrotropes, pH modifiers, surface modifiers, and skin protectants. When employed, miscellaneous additives desirably comprise less than about 2% of the slurry and still more desirably less than about 1% of the slurry and even less than about 0.5% of the slurry.

Formation of the Absorbent Composite

Formation of Highly-Expanded Foam

The foam precursor or slurry is acted upon to form a highly-expanded foam. More specifically, the slurry is acted upon so as to form a highly porous foam having an air content greater than about 50% by volume and desirably an air content greater than about 60% by volume. In certain aspects, the highly-expanded foam is formed having an air content of between about 60% and about 95% and in further aspects between about 65% and about 85%. In certain embodiments, the highly-expanded foam may be acted upon to introduce air bubbles such that the ratio of expansion (volume of air to slurry in the expanded stable foam) is greater than 1:1 and in certain embodiments the ratio of air:slurry can be between about 1:1.1 and about 1:20 or between about 1:1.5 and about 1:15 or between about 1:2 and about 1:10 or even between about 1:3 and about 1:5.

The highly-expanded foam can be generated by one or more means known in the art. Examples of suitable methods include, without limitation, aggressive mechanical agitation, and injection of compressed air. Mixing the components through the use of a high-shear, high-speed mixer is particularly well suited for use in the formation of the desired highly-porous foams. Various high-shear mixers are known in the art and believed suitable for use with the present invention. High-shear mixers typically employ a tank holding the foam precursor and/or one or more pipes through which the foam precursor is directed. The high-shear mixers may use a series of screens and/or rotors to work the precursor and cause aggressive mixing of the components and air. In a particular embodiment, a tank is provided having therein one or more rotors or impellors and associated stators. The rotors or impellors are rotated at high speeds in order to cause flow and shear. Air may, for example, be introduced into the tank at various positions or simply drawn in by the action of the mixer. While the specific mixer design may influence the speeds necessary to achieve the desired mixing and shear, in certain embodiments suitable rotor speeds may be greater than about 500 rpm and, for example, be between about 1000 rpm and about 6000 rpm or between about 2000 rpm and about 4000 rpm. In certain embodiments, with respect to rotor based high-shear mixers, the mixer maybe run with the foam slurry until the disappearance of the vortex in the slurry.

In addition, it is noted the foaming process can be accomplished in a single foam generation step or in sequential foam generation steps. For example, in one embodiment, all of the components may be mixed together to form a slurry from which a highly-expanded foam is formed. Alternatively, one or more of the individual components may be added to the foaming fluid, an initial mixture formed (e.g. a dispersion or foam), after which the remaining components may be added to the initially foamed slurry and then all of the components acted upon to form the final highly-expanded foam. In this regard, the water and foaming surfactant may be initially mixed and acted upon to form an initial foam prior to the addition of any solids. The cellulosic fibers may then be added to the water/surfactant foam and then further acted upon to form the final highly-expanded foam. As a further alternative, the water and fibers may be aggressively mixed to form an initial dispersion after which the foaming surfactant and other components are added to form a second mixture which is then mixed and acted upon to form the highly-expanded foam.

Absorbent Composite Formation

Sheet Formation

The highly-expanded foam, once formed, can be acted upon so as to remove the foaming fluid (e.g. water) without substantial loss or degradation of the porous structure and so as to form an ultra-low density composite cellulosic web. Typically, this will comprise drying of the highly-expanded foam in a non-compressive manner. The highly-expanded foam can be dried by one or more means known in the art such as, for example, heating by radiation such as microwaves or infrared radiation, inductively heated, heated by conduction or convection, such as by gentle (non-compressive) through-air drying, or the like. As an example, and with respect to through-air drying, the highly-expanded foam can be fed onto a foraminous fabric or screen (i.e. a forming fabric) and hot, dry air directed though the foam. In this regard, desirable flow rates are sufficiently low so as not to cause significant compression and/or degradation of the air bubbles strewn throughout the material. Alternatively, a sheet of high-expanded foam can be placed into a convection oven and heated to the desired degree. It will be appreciated that temperatures used in the drying operation should not be so high as to excessively melt any binder fiber that may be present nor so high as to cause significant loss of air bubbles within the material. The foam is desirably dried to achieve a composite cellulosic web wherein less than about 10% of the foaming liquid remains. In certain embodiments, the dried composite cellulosic web will have a water content of between about 1% and about 8% or further between about 2% and 5%.

In one aspect, the highly-expanded foam may be directed onto and spread across a forming fabric or mold to form a sheet-like structure. The spread and height of the foam will be selected in relation and/or proportional to the desired dimensions of the dried cellulosic web.

Absorbent Composite

Once dried, the composite cellulosic material or sheet of the present invention will have very low densities, specifically between 0.04 g/cc and 0.005 g/cc, such as between about 0.02 g/cc and about 0.005 g/cc or even between about 0.099 g/cc and about 0.005 g/cc. Relatedly, in certain aspects, the composite cellulosic web or sheet can comprise a highly porous structure having network of inter-connected pores extending through the thickness of the sheet. In certain embodiments, a significant number and/or majority of pores will have a spherical shape.

The composition of the absorbent composite material can vary in numerous respects to achieve the desired function and/or properties. In certain embodiments, the absorbent composite may comprise the following:

  i. between about 10% to 40% or between about 20% to 40% water resistant fibers;
  ii. between 30 to 45% by wt. cellulosic fibers;
  iii. between about 10 % to about 30% or between about 15% to about 30% binder;
  iv. less than about 5% by wt. foaming surfactant, including for example comprising between about 0.01% to about 5%, or between about 0.05% to about 3% or between about 0.5% to about 2% foaming surfactant;
  v. (when used) less than about 2% by wt. foam stabilizer, including for example comprising between about 0.01% to about 2%, or between about 0.05% to about 2% foam stabilizer;

vi. (when used) less than about 5% by wt. strength enhancing agent, including for example comprising between about 0.05% to about 3%, or between about 0.1% to about 2% strength enhancing agent;

vii. (when used) less than about 45% by wt. SAM, including for example comprising between about 1% to about 45% or between about 10% to about 40% SAM.

Depending on the method of drying the foam, a portion of the foaming surfactant may have been removed with the water. However, regardless of the drying method, an amount of foaming surfactant will remain on the fibers and within the foamed absorbent composite material.

More specifically, despite including significant portions of cellulosic fibers such as wood pulp and having very low densities, the absorbent composite has a Wet Caliper Retention greater than 40%, such as greater than 50% or even greater than 60%. In certain embodiments, absorbent composite can have a Wet Caliper Retention less than about 98%, less than about 95% or even less than about 90%. In further particular embodiments, the ultra-low density cellulosic webs of the present invention can have a Wet Caliper Retention between about 50 to about 99%, between about 60 and about 99% or between about 85 and 99%.

The thickness and basis weight of the sheet can be selected as desired to fulfill the needs of the particular personal care article or other absorbent article incorporating the same. In this regard, the composite cellulosic web or sheet can have a thickness selected to provide the desired degree of absorbency and/or bulk, and in certain aspects can have a thickness between about 0.5 and about 45 mm or, in alternate embodiments, between about 1 mm and 30 mm. The composite cellulosic web or sheet may be converted into a wiping article, absorbent article (such as a personal care article) or a component of such articles. The size of the composite cellulosic web or sheet may be shaped and/or sized as needed to provide sufficient bulk, surface area and/or perform the intended function either alone or as a component in a multi-component article.

Wiping Articles

With respect to uses as a wiping article such as, for example, as a facial tissue, cosmetic wipe, toilet tissue, table napkin, or other wiping article, the cellulosic tissue sheet can have a thickness of between about 0.5 mm and about 5 mm or, in other embodiments between about 1 mm about 3 mm. In certain embodiments, for wiping or cleaning applications, the composite cellulosic sheet can have a basis weight of at least about 10 gsm such as for example, having a basis weight of between about 10 gsm and about 100 gsm or between about 15 gsm and about 90 gsm or even between about 20 gsm and about 80 gsm.

With respect to a wiping and/or cleaning product, the size of the article may be shaped and/or sized to provide sufficient surface area to enable a user to clean and/or treat the intended body part(s). By way of example, for many personal care applications it will be adequate for the article to have a maximum diameter of between about 5 - 45 cm or, in other embodiments, between about 7 and about 35 cm or between about 10 cm and about 30 cm. For certain personal care products, including use as a facial cleansing and/or cosmetic product, the article desirably has a length of between 5 - 30 cm and a width between about 5 - 30 cm, including for example, having dimensions between 25 cm (L) x 25 cm (W) and ( 5 cm (L) $\times$ 5 cm (W). The shape of the article may vary as desired and may comprise rectilinear, curvilinear and irregular shapes. By way of example, the article may be circular, elliptical, oval, square, rectangular, and multi-lobal.

Wiping and/or cleaning articles of the present invention can, in certain aspects, comprise multi-ply products wherein at least one of the layers comprises the ultra-low density composite cellulosic web described herein. The individual layers may, in one aspect, be separately formed and then attached to one another by one or more means known in the art such as, for example, by adhesives, "crimp" or compressive bonding (e.g. application of pressure), or thermal bonding (e.g. where a layer includes one or more thermoplastic binder fibers). In one aspect, the multi-ply webs may be pattern embossed. Such embossing and/or plying techniques are known in the art and examples include, but are not limited to, those described in US3867225 Nystrand; US4320162 to Schulz; and USD305181 to Veith. In addition and/or alternatively, the ultra-low density composite cellulosic web may be formed directly on or with a second layer to form an integrated and/or coherent multi-layer laminate.

In certain aspects, the tissue product may comprise a multi-layer or multi-ply product comprising a first tissue web comprising an ultra-low density composite cellulosic web as described herein and a second paper tissue web having one or more different properties from that of the ultra-low density paper tissue web. In one aspect, the second paper tissue web can have a density of between 0.5 g/cc and 0.04 g/cc or, in further embodiments, a density between about 0.3 g/cc and about 0.05 g/cc. Further, the second tissue sheet can have a basis weight between about 10 to about 100 gsm or between about 20 and about 90 gsm. For certain facial tissue products, each of the layers can have a basis weight between about 10 to about 20 gsm. However, for heavier duty paper products such as a paper towel, in certain embodiments, each of the layers may have a basis weight between about 15 gsm and about 40 gsm. The thickness of the sheets may, in certain embodiments, be between about 0.5 mm and about 5 mm or, in other embodiments, between about 1 mm about 3 mm. The second ply can, in certain embodiments, comprise at least about 95% cellulosic fibers, at least about 98% cellulosic fibers or at least about 99% cellulosic fibers. Further, the fibers making up the second ply can, in certain embodiments, comprise entirely (100%) cellulosic fibers. The second ply can, in one aspect, comprise a wet-formed tissue sheet. Wet formed tissue sheets formed by a through-air drying process are well suited for use in conjunction with the foamed tissue sheet of the present invention; such tissue webs are generally characterized by relatively higher bulk and absorbency than convention wet-pressed tissue and in certain embodiments can be formed with a surface texture to improve cleaning efficacy and/or hand. Tissue sheets of this type are described in and formed by methods described in US4191609 to Trokhan; US5443691 to Van Phan et al.; US5672248 to Wendt et al.; US6787000 Burazin et al.; US2015/0176221 Maiden et al., and WO2015/080726 to Burazin et al. Further, in certain embodiments the second web may comprise a wet-formed tissue sheet dried by a wet-press process and which is characterized by relatively higher density and more compact, planar fiber orientation. Such tissue webs are often dried on a Yankee drier and creped on one or both sides to further increase bulk and improve sheet strength. Tissue sheets of this type are described in and formed by methods described in US4894118 Edwards et al.; US4942077 Kessner et al.; US6454904 Hermans et al.; WO2015/066036 to Bradley et al.

In certain embodiments, the multi-ply tissue product of the present invention can comprise an ultra-low density tissue web as described herein attached to a nonwoven web. Nonwoven webs can be formed from many processes, such as, for example, meltblowing processes, spunbonding processes, air-laying processes, coforming processes, bonded carded web processes, hydroentangling processes. By way of non-limiting example, various nonwoven webs and processes for making the same are described in US3849241 to Butin et al., US4340563 to Appel et al., US4443513 to Meitner et al., US4548856 to Ali Kahn et al., US4853281 to Abba et al., US5382400 to Pike et al., US5575874 to Griesbach et al., US6224977 to Kobylivker et al., US6811638 to Close et al., US6946413 to Lange et al., US2004/0192136 to Gusky et al., US2006/0008621 to Gusky et al., US4100324 to Anderson et al., US5350624 to Georger et al., US2011 /151596 to Jackson et al., US3485706 to Evans, US3620903 to Bunting et al., US5009747 Viazmensky et al., US5284703 to Everhart et al., and US6200669 to Marmon et al. In certain aspects the nonwoven webs can have a basis weight of from about 15 gsm to about 240 gsm or, in alternate embodiments, between about 20 - 190 gsm or between about 25 - 90 gsm. In this regard, the fibrous material includes greater than about 25% by weight synthetic fibers and in certain embodiments between about 40% - 100% synthetic fibers and in still further embodiments between about 55% - 100% synthetic fibers. The synthetic fibers may comprise polymers such as, for example, polyolefin, polyester, polyamide, polylactic acid, or other fiber forming polymers.

In a further aspect, a multi-ply product of the present invention can comprise an ultra-low density composite cellulosic product as described herein plied to a nonwoven web of cellulosic synthetic fibers such as, for example, viscose. In one aspect, a multi-ply product can comprise an ultra-low density tissue product described herein attached to a biodegradable and/or flushable nonwoven web. Examples of biodegradable and/or flushable nonwoven fabrics include, but are not limited to, those described in US5948710 to Pomplun et al., US5667635 Win et al., US6043317 to Mumick et al., WO2007/110497 to Noelle, US2011/0290437 to Vogel et al., and US2015/118466 to Zwick et al.

In certain embodiments, the ultra-low density composite cellulosic web described herein may comprise an outer layer or ply in the multi-ply laminate. In alternate embodiments, the multi-layer tissue product may comprise 3 or more layers. In certain such embodiments and in relation to a multi-ply web having 3 or more plies, the ultra-low density composite cellulosic web described herein may comprise an inner or central ply and wherein first and second outer plies can comprise wet-laid paper tissue webs or nonwoven webs described herein above. Further, in such embodiments the first and second outer plies may be the same or different materials.

The wiping products or sheets, comprising or incorporating the absorbent composite materials described herein, can be provided in a stack and incorporated or oriented in a container as desired. Further, the stack of sheets can be folded as desired in order to improve efficiency of use and/or dispensing. The stack(s) of the wiping products are desirably arranged to facilitate removal or dispensing, and in certain embodiments, can be arranged to facilitate metered dispensing. The sheets may be inter-folded in various know overlapping configurations such as, for example, V-folds, Z-folds, W-folds, and quarter-folds. Equipment and processes for forming dispensable stacks of wipes are known in the art; examples of which include, but are not limited to, those described in US3401927 to Frick et al.; US4502675 to Clark et al.; US5310398 to Onekama, and US6612462 to Sosalla et al. With respect to product formats utilizing a continuous length of sheet material, such as in certain rolled formats, the individually separable wet wipes desirably have perforated or over-bonded lines of weakness which allow separation into smaller individual sheets of a desired shape and size. The particular stack height and sheet count can vary with the intended format and use. However, in certain embodiments, the stack may include between 3 and 250 individual wiping products or sheets, in further embodiments may include between about 10 and 150 wiping products or sheets and in still further embodiments may include between about 10 and 90 wiping products or sheets.

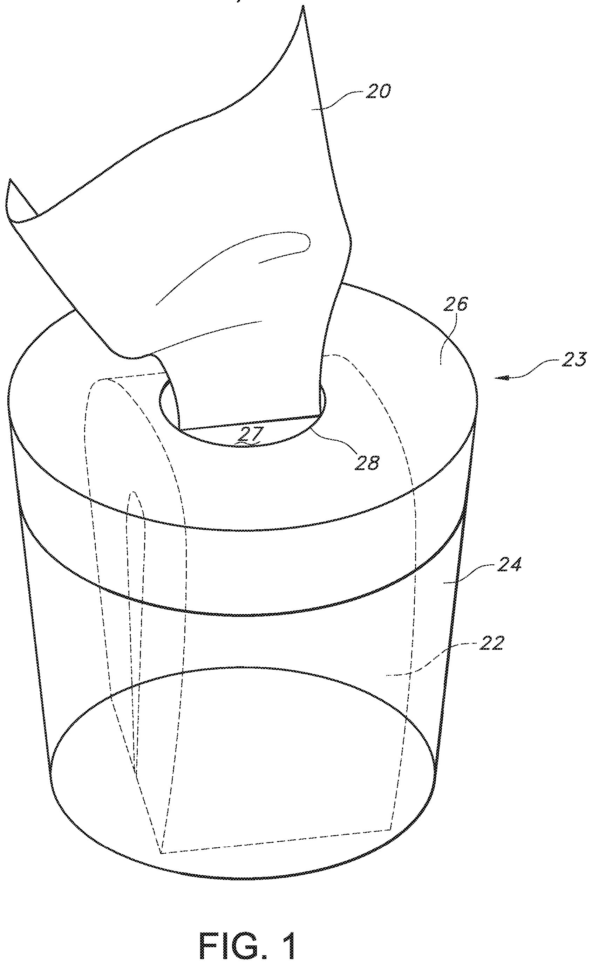

The stack of wiping products may be housed within a container adapted for the maintenance and/or dispensing of the same. Numerous such containers, e.g. packages and/or dispensers, are known in the art and suitable for use herewith. In one aspect, the container includes a plurality of walls defining an interior space. In one aspect, the container may comprise a flexible pack formed by thin polymeric film and optionally further having a resealable or reclosable dispensing opening for accessing the wiping products contained therein. Examples of flexible packaging dispensers include, but are not limited to, those described in US4863064 to Dailey, US5524759 Herzberg et al., US6012572 Heathcock et al., US7527152 to House et al., WO96/37138 Bauer et al., and US2010/155284 Foley et al. In addition, substantially rigid dispensers suitable for dispensing the paper tissue products of the present invention include, but are not limited to, those described in US3369700 Nelson et al., US3369699 Enloe et al., US5785179 Buczwinski et al., US7028840 Huang et al., US7543423 Long et al., US2005/211718 Decker et al., and US2007/0235466 Fulscher et al. In a particular embodiment, such as seen in reference to Figure 1, a stack 22 of individual wiping products or sheets 20 may be positioned within a container or dispenser 24. The sheet stack 22 is located within the walls 24 of the dispenser 23 and may comprise the substantial majority of the interior space of the dispenser 23. The dispenser 23 can have at least one side panel, such as a top side panel 26, having a dispensing opening 28 through which the individual sheets 20 can be removed. Commonly, the dispensing opening 28 will have a tab or cover (not shown) that is positioned over the dispensing opening but which is designed to be removed by the ultimate user at the time of use. To facilitate one at a time or metered dispensing, the dispensing opening 28 can have a flexible membrane or film 27 partially occluding the dispensing opening and designed to provide a frictional force on the sheet 20 being removed and thereby facilitate proper separation of the individual sheet 20 from the stack 22.

Personal Care Absorbent Articles

Absorbent personal care articles generally include a liquid permeable topsheet, which faces the wearer, and a liquid-impermeable backsheet or outer cover. Disposed between the topsheet and outer cover is an absorbent core. In this regard, the topsheet and outer cover are often joined and/or sealed to encase the absorbent core. Although certain aspects of the present invention are described in the context of a particular personal care absorbent article, it will be readily appreciated that similar uses in other types of personal care absorbent articles and/or further combinations or alterations of the specific configurations discussed below may be made by one skilled in the art without departing from the spirit and scope of the present invention.

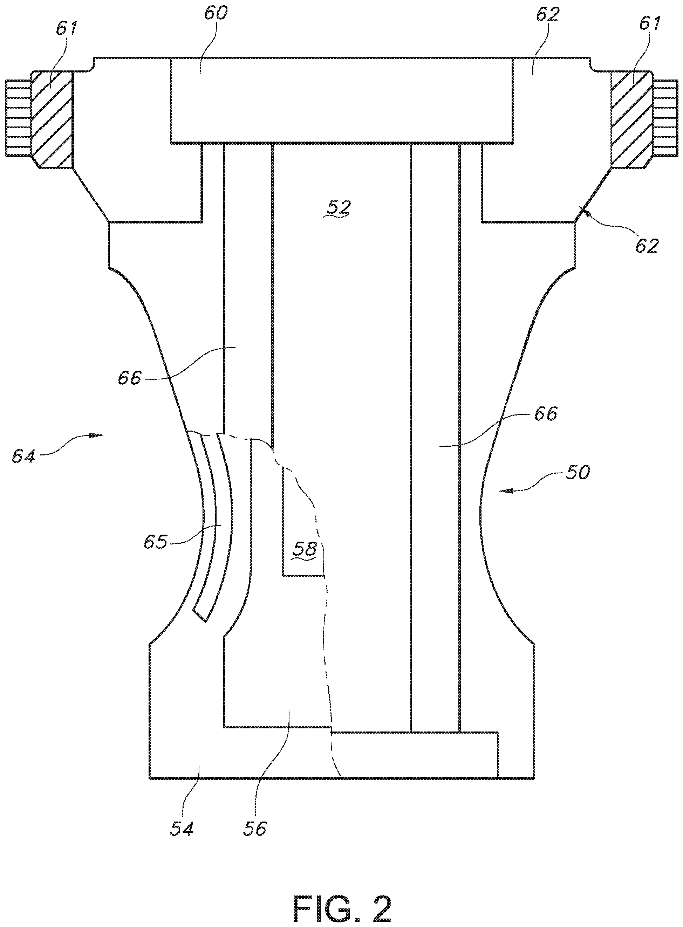

In a particular embodiment, and in reference to Fig. 2, a diaper 50 can comprise a liquid-impervious outer cover 54, a liquid permeable topsheet 52 positioned in facing relation to the outer cover 54, and an absorbent core 56 between the outer cover 54 and topsheet 52. The diaper 50 may be of various shapes such as, for example, an overall rectangular shape, a T-shape, or an hourglass shape. The topsheet 52 is generally coextensive with the outer cover 54 but may optionally cover an area that is larger or smaller than the area of the outer cover 54, as desired. While not shown, it is to be understood that portions of the diaper, such as a marginal section of the outer cover, may extend past and around the terminal edges of the product and form a portion of the body-facing layer. The diaper 50 also has end margins 62 adapted to fit about the waist of the wearer and a central region 64 designed to extend about the groin region as well as provide leg openings in combination with end margins 62. Fastening members 61 are commonly located adjacent the end margins 62 for securing the diaper about the wearer.

The topsheet or body-side liner 52, as representatively illustrated in Fig. 2, desirably presents a body facing surface which is compliant, soft to the touch, and non-irritating to the wearer's skin. The topsheet 52 is desirably employed to help isolate the wearer's skin from liquids held in the absorbent core 56. Topsheets are well known in the art and may be manufactured from a wide variety of materials, such as porous foams, reticulated foams, apertured plastic films, natural fibers (wool, cotton fibers, etc.), synthetic fibers (polyester, polypropylene, polyethylene, etc.), and combinations of natural and synthetic fibers. Topsheets can comprise a single layer or multiple layers including a combination of one or more different materials. Nonwoven fabrics, and laminates thereof, are commonly utilized to form topsheets. Suitable topsheet materials include, but not limited to, those described in US5382400 to Pike et al.; US5415640 to Kirby et al.; US5527300 to Sauer; US5994615 to Dodge et al.; US6383960 to Everett et al.; US6410823 to Daley et al.; and US2014/0121623 to Biggs et al.

The backsheet or outer cover 54 comprises a liquid-impervious material. Desirably, the outer cover comprises a material that prevents the passage of water but allows air and water-vapor to pass there through. The outer cover can comprise a single layer of material or multiple layers including one or more layers of different materials. In a particular embodiment, the outer cover can comprise a film fixedly attached or bonded to one or more nonwoven webs. The particular structure and composition of the outer cover may be selected from various combinations of films and/or fabrics. In this regard, the outer most layers are generally selected for providing the desired strength, abrasion resistance, tactile properties and/or aesthetics. As an example, nonwoven fabrics are commonly employed as the outermost layer of the outer cover. Suitable outer covers include, but are not limited to, those described in US4041203 to Brock et al.; US6075179 et al. to McCormack et al.; US6111163 to McCormack s et al.; and US2015/099086 to Cho et al.

Between the liquid-impervious outer cover 54 and the liquid pervious topsheet 52 is positioned an absorbent core 56. The absorbent core may itself comprise a multiple layers having similar or different absorbent properties. In this regard, the absorbent core often includes one or more absorbent materials including, but not limited to, superabsorbent particles, wood pulp fibers, synthetic wood pulp fibers, synthetic fibers and combinations thereof. The absorbent core may have any of a number of shapes and the size of the absorbent core and selection of materials therein will vary with the desired loading capacity, the intended use of the absorbent article and other factors known to those skilled in the art. Examples of various absorbent core structures and/or additional absorbent materials that may be used in conjunction with the present invention include, but are not limited to, those described in US4699823 to Kellenberger et al.; US5458592 to Abuto et al; US5601542 to Melius et al.; US6129720 to Blenke et al.; US6383960 to Everett et al.; US7938813 to Elliker et al.; and US2002/0183703 Dodge et al. In one aspect, the absorbent core may comprise one or more layers, wherein at least one layer comprises the ultra-low density composite cellulosic web or sheet described herein. Further, in certain embodiments, the absorbent core may include a plurality of superposed ultra-low density cellulosic composite materials described herein.

In addition, it is also common for the personal care absorbent article to employ a liquid distribution layer or "wicking" layer 58 above the absorbent core 56 in order to increase the span and/or regulate the speed with which the liquid coming into the absorbent article reaches the absorbent core. However, in other embodiments, the liquid transfer layer may alternatively and/or additionally be placed beneath the absorbent core (not shown). Such materials can comprise one or more nonwoven webs including for example, but not limited to multi-functional air-laid materials, apertured film/nonwoven laminates, and high-loft nonwoven fabrics. Examples of various liquid distribution layers include, but are not limited to, those described in US5364382 to Latimer et al.; US6437214 to Bolwerk et al.; US6534149 Mace et al.; and US6617490 Lindsay et al. In certain embodiments, the absorbent personal care article may include a liquid distribution layer comprising an ultra-low density cellulosic composite material described herein.

The personal care articles can, optionally, contain one or more additional elements or components. In this regard, numerous additional features and various constructions are known in the art. As but a few examples and in reference to FIG. 2, the diaper can further include elastic leg cuffs 65, an elastic waistband 60, and fluid control flaps 66. One skilled in the art will appreciate the application and use of the ultra-low density webs of the present invention in like manners for other absorbent personal care articles including, for example, adult incontinence garments, incontinence pads/liners, sanitary napkins, party-liners, sweat pads, and bandages. In addition, other absorbent articles that may include one or more layers of the ultra-low density webs of the present invention including, but are not limited to, baby bibs and changing pads, bed pads, and food tray liners.

The ultra-low density composite cellulosic webs and products can, optionally, include one or more additional elements or components as are known in the art. Thus, while the invention has been described in detail with respect to specific embodiments and/or examples thereof, it will be apparent to those skilled in the art that various alterations, modifications and other changes may be made to the invention without departing from the spirit and scope of the same. It is therefore intended that the claims cover or encompass all such modifications, alterations and/or changes.

**Definitions and Test Methods**

In reference to the Figures and throughout the specification and claims, discussion of the articles and/or individual components thereof is with the following understanding:

The terms "comprising" or "including" or "having" are inclusive or open-ended and do not exclude additional unrecited elements, compositional components, or method steps. Accordingly, the terms "comprising" or "including" or "having" also encompass the more restrictive terms "consisting essentially of and "consisting of".

As used herein, the term "basis weight" generally refers to the bone dry weight per unit area of a tissue or web and is generally expressed as grams per square meter (gsm). Basis weight is measured using TAPPI test method T-220 sp-10.

As used herein, the term "nonwoven web" means a web having a structure of individual fibers or threads which are interlaid, but not in an identifiable manner as in a knitted web.

The term "personal care absorbent article" refers herein to an article intended and/or adapted to be placed against or in proximity to the body (i.e., contiguous with the body) of the wearer to absorb and contain various liquid, solid, and semi-solid exudates discharged from the body. Examples include, but are not limited to, diapers, diaper pants, training pants, youth pants, swim pants, feminine hygiene products, including, but not limited to, menstrual pads or pants, incontinence products, medical garments, surgical pads and bandages.

The term "superabsorbent polymer" as used herein refers to water-swellable, water-insoluble organic or inorganic materials including superabsorbent polymers and superabsorbent polymer compositions capable, under the most favorable conditions, of absorbing at least about 10 times their weight, or at least about 15 times their weight, or at least about 25 times their weight in an aqueous solution containing 0.9 weight percent sodium chloride.

The term "ply" refers to a discrete layer within a multi-layered product wherein individual plies may be arranged in juxtaposition to each other.

The term "plied" or "bonded" or "coupled" refers herein to the joining, adhering, connecting, attaching, or the like, of two elements. Two elements will be considered plied, bonded or coupled together when they are joined, adhered, connected, attached, or the like, directly to one another or indirectly to one another, such as when each is directly bonded to intermediate elements. The plying, bonding or coupling of one element to another can occur via continuous or intermittent bonds.

As used herein, unless expressly indicated otherwise, when used in relation to material compositions the terms "percent", "percent", "weight percent" or "percent by weight" each refer to the quantity by weight of a component as a percentage of the total except as whether expressly noted otherwise.

As used herein, the term "stack" is used broadly to include any collection of sheets wherein there is a plurality of individual sheets having surface-to-surface interfaces; this not only includes a vertically stacked collection of individual sheets, but also includes a horizontally stacked collection of individual sheets as well as a rolled or folded collection of continuous sheet material separated by lines of weakness (e.g. perforations).

As used herein "caliper" or "thickness" of a sheet is determined by using a micrometer having an acrylic platen with a pressure foot area of 45.6 cm2 (3 inch diameter), providing a load of 0.345 kPa (0.5 psi), and the reading is taken after a dwell time of 3 seconds. A sample is cut having a size of $90 \times 102$ mm ($3.5 \times 4$ inches) is used for the measurement. Unless expressly stated otherwise, reference to caliper or thickness is the dry caliper. Wet caliper is measured by taking the material treated and measured for the dry caliper reading and then saturating the sample in deionized water. The wet sample is then dried and after drying measured to determine caliper in the same method as above for the dry caliper.

"Wet Caliper Retention" means and is calculated according to the following formula: (Wet Caliper $\div$ Dry Caliper) x 100

As used herein "Water retention value" or "WVR" of fibers determines the absorbency and water retention properties of a particular type of fiber and is determined as follows. The sample fibers are selected and 0.7 g dry fibers are weighed and placed into 100 mL purified water for four hours. The soaked fibers are then placed in a centrifuge and subjected to a centrifugal acceleration equal to 900 times the force of gravity for 30 minutes. The fiber sample is removed and then weighed. WRV = (wet weight - dry weight)/dry weight.

## Examples

Examples 1-5 were made according to the following process. A total of 2000 grams of an initial slurry was added into a disintegrator's cup for fiberizing wood pulp fiber. The initial slurry was formed to having a fiber consistency of between 3-5%. 40 grams of dry commercial densified wood pulp sheet and, where applicable additional fibers, were weighed according to fiber mixture weight ratio and placed into the British standard disintegrator's cup. 1960 grams of distilled water was added into the cup and the fiber mixture was soaked for 5 minutes. The cup was then mounted onto the disintegrator and ran for 15,000 revolutions to sufficiently fiberize the wood pulp fiber. After this, 500 grams of the initial slurry was weighed in a separate container having a dimension of 150 mm in diameter and 170 mm in height and 6.5 grams of 10% sodium dodecyl sulfate solution was added into the container. The surfactant concentration in the slurry was about 0.13 weight percent. The slurry was mixed by a high speed rotary mixer at a speed of 4000 rpm for several minutes until the vertex inside the container disappeared.

The foam formed fiber slurry was then shaped into a sheet by two different ways. Method 1 was to pour entire foamed slurry into a lab hand sheet former with an area dimension of 10 inches by 10 inches. Method 2 was to control thickness of the wet foam sheet by pushing it through a gap with the gap height controlled through the use of different diameter metal rods.

The foam sheets were dried in an oven at 90°C for about 2 to 3 hours. The dried base sheets were subsequently heated at a temperature suitable to activate binder fiber and achieve thermo-bonding as between the binder fiber and other fibers contacting the same.

Example 1 (reference): The slurry used to form the expanded foam included the SDS as noted above and the solids consisted of 50% Eucalyptus wood pulp fiber, 30% PET staple fiber with a fiber length of 6 mm, and 20% Trevira T255-6 polyethylene/PET sheath/core staple fiber with a 6 mm fiber length and 2.2 dtex.

Example 2 (reference): The slurry used to form the expanded foam included the SDS as noted above and the solids consisted of 60% LL 19 northern softwood kraft fiber (LL-19) and 40% Trevira T255-6 polyethylene/PET sheath/core staple fiber with a 6 mm fiber length and 2.2 dtex.

Example 3: The slurry used to form the expanded foam included the SDS as noted above and the solids consisted of 32% Eucalyptus wood pulp fiber, 32% 6 mm PET staple fiber, 20% 6 mm melamine staple fiber having an average fiber diameter of about 20 micrometers, and 16% Trevira T255-6 polyethylene/PET sheath/core staple fiber with a 6 mm fiber length and 2.2 dtex.

Example 4: The slurry used to form the expanded foam included the SDS as noted above and the solids consisted of 35% Eucalyptus wood pulp fiber, 6 mm PET staple fiber, and 30% Trevira T255-6 polyethylene/PET sheath/core staple fiber with a 6 mm fiber length and 2.2 dtex.

Example 5: The slurry used to form the expanded foam included the SDS as noted above and the solids consisted of 35% cotton flock, 35% Evonik SSM 5600 superabsorbent particles predominantly having particle sin the size range of 300-600 micrometers, and 30% Trevira T255-6 polyethylene/PET sheath/core staple fiber with a 6 mm fiber length and 2.2 dtex.

Table 1 A - Summary of Properties of Examples 1-5

| | Consistency (% Solids by Wt. in Slurry) | Dry Density (g/cc) | Dry Caliper (mm) | Wet & Dried Caliper (mm) | % Wet Caliper Retention |
|---|---|---|---|---|---|
| 1 (reference) | 3% | 0.0111 | 4.53 | 2.01 | 44.4 |
| 2 (reference) | 4% | 0.0166 | 7.01 | 5.62 | 80.2 |
| 3 | 5% | 0.0156 | 3.79 | 3.71 | 97.9 |
| 4 | 3% | 0.0131 | 6.87 | 6.55 | 95.3 |
| 5 | 3% | 0.029 | 8.8 | 8.06 | 91.6 |

**Claims**

1. An absorbent composite foam comprising:

   between 5 to 40% by wt. water resistant fibers;
   between 30 to 45% by wt. cellulosic fibers;
   between 5 to 35% by wt. binder;
   a foaming surfactant; and
   wherein binder bonds adjacent fibers at fiber contact points and said cellulosic fibers are bonded to one another by means of hydrogen bonding, and further wherein the absorbent composite has a density between 0.04 g/cc and 0.005 g/cc and a Wet Caliper Retention of greater than 40%, wherein the Wet Caliper Retention is determined according to the method provided in the description herein.

2. The absorbent composite foam of claim 1 wherein at least a majority of the cellulosic fibers comprise wood pulp fibers.

3. The absorbent composite foam of claim 1 wherein said foam has a dry caliper between 0.5 and 40 mm.

4. The absorbent composite foam of claims 1 wherein said foam has a thickness of between 0.5 mm and 5 mm and has a substantially planar, sheet-like structure.

5. The absorbent composite foam of a claim 1 wherein

   said water resistant fibers comprise between 20 to 40% of the composite foam; and
   said binder comprises binder fibers and comprises between 15 to 35% of the composite foam.

6. The absorbent composite foam of claim 1 wherein said foaming agent is selected from cationic and anionic surfactants and is present in said foam between 0.05 and 5%; or wherein said water resistant fibers comprise polymeric fibers selected from the group consisting of polyolefin, polyester, polyamide, and polylactic acid polymers; or further comprising a wet strength additive associated with said cellulosic fibers.

7. The absorbent composite foam of claim 3 further comprising a superabsorbent material.

8. The absorbent composite foam of claim 1 or 5 wherein the density of said foam is between 0.02 g/cc and 0.005 g/cc and further wherein said foam has a wet retention value in excess of 60%.

9. A multi-layer laminate comprising:

   a first layer comprising the absorbent composite foam of claim 1;
   a second layer comprising a nonwoven web selected from the group consisting of wet-laid webs, air-laid webs, and hydroentangled webs; wherein said nonwoven web preferably comprises a paper tissue web having a density of between 0.5 g/cc and 0.04 g/cc and wherein said paper tissue web comprises at least 95% cellulosic fibers.

10. The multi-layer laminate of claim 9 wherein the first and second layers are pattern embossed.

11. The multi-layer laminate of claim 9 further comprising a third layer comprising at least 98% wood pulp fibers and wherein said first layer is positioned between said second and third layers.

12. The multi-layer laminate of claim 9 wherein said nonwoven web comprises a nonwoven web selected from the group consisting of meltblown fiber webs, spunbond fiber webs, hydroentangled webs and air-laid fiber webs and further wherein the air-laid fibers and have a basis weight between 12 gsm and 70 gsm.

13. A packaged wiping product comprising:

   a container defining an interior space and having a resealable dispensing opening;
   a stack of between 3 and 150 individual wiping sheets, said stack located within said interior space and said wiping sheets comprising the absorbent composite foam of claim 1.

14. The packaged wiping product of claim 13 wherein said wiping sheets comprise a multi-ply sheet and further wherein the absorbent composite foam has a sheet-like structure having a thickness of between 0.5 and 5 mm and a density of between 0.02 g/cc and 0.005 g/cc.

15. The absorbent composite foam of claim 1, wherein the binder is thermoplastic binder fibers.

16. A personal care absorbent article comprising:

   a liquid pervious body-side liner;
   a liquid impervious outer cover;
   an absorbent core, said absorbent core is located between and said body-side liner and said outer cover; and wherein the absorbent core includes the absorbent composite foam of claim 3.

17. A personal care absorbent article comprising:

   a liquid pervious body-side liner;
   a liquid impervious outer cover;
   an absorbent core, said absorbent core is located between and said body-side liner and said outer cover; and
   a liquid transfer layer superposed with said absorbent core and located between and said body-side liner and said outer cover; and
   wherein the liquid transfer layer includes the absorbent composite foam of claim 4.

**Patentansprüche**

1. Absorbierender Verbundschaum, umfassend:

   zwischen 5 und 40 Gew.-% wasserbeständige Fasern;

zwischen 30 und 45 Gew.-% Cellulosefasern;
zwischen 5 und 35 Gew.-% Bindemittel;
ein schäumendes Tensid; und
wobei das Bindemittel benachbarte Fasern an Faserkontaktpunkten bindet und die Cellulosefasern mittels Wasserstoffbindung miteinander verbunden sind, und wobei der absorbierende Verbundstoff ferner eine Dichte zwischen 0,04 g/cm$^3$ und 0,005 g/cm$^3$ und ein Nassdicke-Rückhaltevermögen von mehr als 40 % aufweist, wobei das Nassdicke-Rückhaltevermögen gemäß dem in der Beschreibung hierin bereitgestellten Verfahren ermittelt wird.

2. Absorbierender Verbundschaum nach Anspruch 1, wobei zumindest eine Mehrheit der Cellulosefasern Holzzellstofffasern umfasst.

3. Absorbierender Verbundschaum nach Anspruch 1, wobei der Schaum eine Trockendicke zwischen 0,5 und 40 mm aufweist.

4. Absorbierender Verbundschaum nach Anspruch 1, wobei der Schaum eine Dicke zwischen 0,5 mm und 5 mm aufweist und eine im Wesentlichen ebene, blattartige Struktur aufweist.

5. Absorbierender Verbundschaum nach Anspruch 1, wobei

die wasserbeständigen Fasern zwischen 20 und 40 % des Verbundschaums ausmachen; und
das Bindemittel Bindefasern umfasst und zwischen 15 bis 35 % des Verbundschaums ausmacht.

6. Absorbierender Verbundschaum nach Anspruch 1, wobei das Schäummittel ausgewählt ist aus kationischen und anionischen Tensiden und in dem Schaum zwischen 0,05 und 5 % vorliegt; oder wobei die wasserbeständigen Fasern Polymerfasern umfassen, die ausgewählt sind aus der Gruppe bestehend aus Polyolefin-, Polyester-, Polyamid- und Polymilchsäurepolymeren; oder ferner umfassend einen Nassfestigkeitszusatz, der mit den Cellulosefasern verbunden ist.

7. Absorbierender Verbundschaum nach Anspruch 3, der ferner ein superabsorbierendes Material umfasst.

8. Absorbierender Verbundschaum nach Anspruch 1 oder 5, wobei die Dichte des Schaums zwischen 0,02 g/cm$^3$ und 0,005 g/cm$^3$ beträgt, und wobei der Schaum ferner einen Nassrückhaltevermögenswert von mehr als 60 % aufweist.

9. Mehrschichtlaminat, umfassend:

eine erste Schicht, die den absorbierenden Verbundschaum nach Anspruch 1 umfasst;
eine zweite Schicht, die eine Vliesbahn umfasst, die ausgewählt ist aus der Gruppe bestehend aus nassgelegten Bahnen, luftgelegten Bahnen und hydroverschlungenen Bahnen; wobei die Vliesbahn vorzugsweise eine Papiergewebebahn mit einer Dichte zwischen 0,5 g/cm$^3$ und 0,04 g/cm$^3$ umfasst, und wobei die Papiergewebebahn mindestens 95 % Cellulosefasern umfasst.

10. Mehrschichtlaminat nach Anspruch 9, wobei die erste und die zweite Schicht eine Musterprägung aufweisen.

11. Mehrschichtlaminat nach Anspruch 9, das ferner eine dritte Schicht umfasst, die mindestens 98 % Holzzellstofffasern umfasst, und wobei die erste Schicht zwischen der zweiten und der dritten Schicht positioniert ist.

12. Mehrschichtlaminat nach Anspruch 9, wobei die Vliesbahn eine Vliesbahn umfasst, die ausgewählt ist aus der Gruppe bestehend aus schmelzgeblasenen Faserbahnen, Spinnvliesfaserbahnen, hydroverschlungenen Bahnen und luftgelegten Faserbahnen, und wobei die luftgelegten Fasern ferner ein Flächengewicht zwischen 12 g/m$^2$ und 70 g/m$^2$ aufweisen.

13. Verpacktes Wischprodukt, umfassend:

einen Behälter, der einen Innenraum definiert und eine wiederverschließbare Ausgabeöffnung aufweist;
einen Stapel von zwischen 3 und 150 einzelnen Wischtüchern, wobei der Stapel innerhalb des Innenraums angeordnet ist und die Wischtücher den absorbierenden Verbundschaum nach Anspruch 1 umfassen.

**14.** Verpacktes Wischprodukt nach Anspruch 13, wobei die Wischtücher ein mehrlagiges Blatt umfassen, und wobei der absorbierende Verbundschaum ferner eine blattartige Struktur mit einer Dicke zwischen 0,5 und 5 mm und einer Dichte zwischen 0,02 g/cm$^3$ und 0,005 g/cm$^3$ aufweist.

**15.** Absorbierender Verbundschaum nach Anspruch 1, wobei es sich bei dem Bindemittel um thermoplastische Bindefasern handelt.

**16.** Absorbierender Körperpflegeartikel, umfassend:

eine flüssigkeitsdurchlässige körperseitige Decklage;
eine flüssigkeitsundurchlässige äußere Deckschicht;
einen absorbierenden Kern, wobei sich der absorbierende Kern zwischen und der körperseitigen Decklage und der äußeren Deckschicht befindet; und
wobei der absorbierende Kern den absorbierenden Verbundschaum nach Anspruch 3 beinhaltet.

**17.** Absorbierender Körperpflegeartikel, umfassend:

eine flüssigkeitsdurchlässige körperseitige Decklage;
eine flüssigkeitsundurchlässige äußere Deckschicht;
einen absorbierenden Kern, wobei sich der absorbierende Kern zwischen und der körperseitigen Decklage und der äußeren Deckschicht befindet; und
eine Flüssigkeitsübertragungsschicht, die mit dem absorbierenden Kern überlagert ist und sich zwischen und der körperseitigen Decklage und der äußeren Deckschicht befindet; und
wobei die Flüssigkeitsübertragungsschicht den absorbierenden Verbundschaum nach Anspruch 4 beinhaltet.

**Revendications**

**1.** Mousse composite absorbante comprenant :

entre 5 à 40 % en poids de fibres résistantes à l'eau ;
entre 30 à 45 % en poids de fibres cellulosiques ;
entre 5 à 35 % en poids de liant ;
un tensioactif moussant ; et
dans laquelle le liant lie des fibres adjacentes au niveau de points de contact de fibres et lesdites fibres cellulosiques sont liées les unes aux autres au moyen d'une liaison hydrogène, et en outre dans laquelle le composite absorbant a une masse volumique entre 0,04 g/cm3 et 0,005 g/cm3 et une rétention de calibre humide supérieure à 40 %, dans lequel la rétention de calibre à l'état humide est déterminée selon le procédé fourni dans la description ici.

**2.** Mousse composite absorbante selon la revendication 1, dans laquelle au moins une majorité des fibres cellulosiques comprennent des fibres de pâte de bois.

**3.** Mousse composite absorbante selon la revendication 1, dans laquelle ladite mousse a un calibre à l'état sec entre 0,5 et 40 mm.

**4.** Mousse composite absorbante selon la revendication 1, dans laquelle ladite mousse a une épaisseur entre 0,5 mm et 5 mm et a une structure sensiblement plane, analogue à une feuille.

**5.** Mousse composite absorbante selon la revendication 1, dans laquelle

lesdites fibres résistantes à l'eau constituent entre 20 à 40 % de la mousse composite ; et
ledit liant est constitué de fibres liantes et constitue entre 15 à 35 % de la mousse composite.

**6.** Mousse composite absorbante selon la revendication 1, dans laquelle ledit agent moussant est choisi parmi des tensioactifs cationiques et anioniques et est présent dans ladite mousse entre 0,05 et 5 % ; ou dans laquelle lesdites fibres résistantes à l'eau sont constituées de fibres polymères choisies dans le groupe constitué par des polymères de polyoléfine, de polyester, de polyamide et de polyacide lactique ; ou comprenant en outre un additif de résistance à l'état humide associé auxdites fibres cellulosiques.

7. Mousse composite absorbante selon la revendication 3, comprenant en outre un matériau superabsorbant.

8. Mousse composite absorbante selon la revendication 1 ou 5, dans laquelle la masse volumique de ladite mousse est comprise entre 0,02 g/cm3 et 0,005 g/cm3 et en outre dans laquelle ladite mousse a une valeur de rétention à l'état humide supérieure à 60 %.

9. Stratifié multicouche comprenant :

une première couche comprenant la mousse composite absorbante de la revendication 1 ;
une deuxième couche comprenant une bande non tissée choisie dans le groupe constitué par des bandes appliquées par voie humide, des bandes appliquées par voie pneumatique et des bandes hydroliées ; dans lequel ladite bande non tissée comprend de préférence une bande de papier mousseline ayant une masse volumique entre 0,5 g/cm3 et 0,04 g/cm3 et dans lequel ladite bande de papier mousseline est constituée d'au moins 95 % de fibres cellulosiques.

10. Stratifié multicouche selon la revendication 9, dans lequel les première et deuxième couches sont gaufrées en motif.

11. Stratifié multicouche selon la revendication 9, comprenant en outre une troisième couche constituée d'au moins 98 % de fibres de pâte de bois et dans lequel ladite première couche est positionnée entre lesdites deuxième et troisième couches.

12. Stratifié multicouche selon la revendication 9, dans lequel ladite bande non tissée comprend une bande non tissée choisie dans le groupe constitué par des bandes de fibres obtenues par fusion-soufflage, des bandes de fibres filées-liées, des bandes hydroliées et des bandes de fibres obtenues par voie pneumatique, et dans lequel en outre les fibres obtenues par voie pneumatique ont un grammage entre 12 g/m$^2$ et 70 g/m$^2$.

13. Produit d'essuyage emballé comprenant :

un contenant définissant un espace intérieur et ayant une ouverture de distribution libérable ;
un empilement de 3 à 150 feuilles d'essuyage individuelles, ledit empilement étant situé au sein dudit espace intérieur et lesdites feuilles d'essuyage comprenant la mousse composite absorbante de la revendication 1.

14. Produit d'essuyage emballé selon la revendication 13, dans lequel lesdites feuilles d'essuyage comprennent une feuille multi-pli et dans lequel en outre la mousse composite absorbante a une structure analogue à une feuille ayant une épaisseur entre 0,5 et 5 mm et une masse volumique entre 0,02 g/cm$^3$ et 0,005 g/cm$^3$.

15. Mousse composite absorbante selon la revendication 1, dans laquelle le liant est composé de fibres de liant thermoplastiques.

16. Article absorbant d'hygiène personnelle comprenant :

un revêtement côté corps perméable aux liquides ;
une enveloppe externe imperméable aux liquides ;
un noyau absorbant, ledit noyau absorbant est situé entre ledit revêtement côté corps et ladite enveloppe externe ; et
dans lequel le noyau absorbant comporte la mousse composite absorbante de la revendication 3.

17. Article absorbant d'hygiène personnelle comprenant :

un revêtement côté corps perméable aux liquides ;
une enveloppe externe imperméable aux liquides ;
un noyau absorbant, ledit noyau absorbant est situé entre ledit revêtement côté corps et ladite enveloppe externe ; et
une couche de transfert de liquide superposée audit noyau absorbant et située entre ledit revêtement côté corps et ladite enveloppe externe ; et
dans lequel la couche de transfert de liquide comporte la mousse composite absorbante de la revendication 4.

FIG. 1

FIG. 2

**EP 3 370 592 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9824621 A
- US 4793898 A, Laamanen
- US 4594130 A, Chang
- US 3585104 A, Kleinhart
- US 5595628 A, Gordon
- US 5522967 A, Shet
- US 4785030 A, Hager
- US 6462159 B, Hamada
- US 6752905 B, Chuang
- US 7396584 B, Azad
- US 7935860 B, Dodge
- US 20055245393 A, Azad
- US 201409606 B, Bergam
- WO 2008027488 A, Chang
- US 3556932 A, Coscia
- US 3556933 A, Williams
- US 6224714 B, Schroeder
- US 6274667 B, Shannon
- US 6287418 B, Schroeder
- US 6365667 B, Shannon
- US 3700623 A, Keim
- US 3772076 A, Keim
- US 3855158 A, Petrovich
- US 3899388 A, Petrovich
- US 4129528 A, Petrovich
- US 4147586 A, Petrovich
- US 4222921 A, van Eenam
- US 3867225 A, Nystrand
- US 4320162 A, Schulz
- US D305181 S, Veith
- US 4191609 A, Trokhan
- US 5443691 A, Van Phan
- US 5672248 A, Wendt
- US 6787000 B, Burazin
- US 20150176221 A, Maiden
- WO 2015080726 A, Burazin
- US 4894118 A, Edwards
- US 4942077 A, Kessner
- US 6454904 B, Hermans
- WO 2015066036 A, Bradley
- US 3849241 A, Butin
- US 4340563 A, Appel
- US 4443513 A, Meitner
- US 4548856 A, Ali Kahn
- US 4853281 A, Abba
- US 5382400 A, Pike
- US 5575874 A, Griesbach
- US 6224977 B, Kobylivker
- US 6811638 B, Close
- US 6946413 B, Lange
- US 20040192136 A, Gusky
- US 20060008621 A, Gusky
- US 4100324 A, Anderson
- US 5350624 A, Georger
- US 2011151596 A, Jackson
- US 3485706 A, Evans
- US 3620903 A, Bunting
- US 5009747 A, Viazmensky
- US 5284703 A, Everhart
- US 6200669 B, Marmon
- US 5948710 A, Pomplun
- US 5667635 A, Win
- US 6043317 A, Mumick
- WO 2007110497 A, Noelle
- US 20110290437 A, Vogel
- US 2015118466 A, Zwick
- US 3401927 A, Frick
- US 4502675 A, Clark
- US 5310398 A, Onekama
- US 6612462 B, Sosalla
- US 4863064 A, Dailey
- US 5524759 A, Herzberg
- US 6012572 A, Heathcock
- US 7527152 B, House
- WO 9637138 A, Bauer
- US 2010155284 A, Foley
- US 3369700 A, Nelson
- US 3369699 A, Enloe
- US 5785179 A, Buczwinski
- US 7028840 B, Huang
- US 7543423 B, Long
- US 2005211718 A, Decker
- US 20070235466 A, Fulscher
- US 5415640 A, Kirby
- US 5527300 A, Sauer
- US 5994615 A, Dodge
- US 6383960 B, Everett
- US 6410823 B, Daley
- US 20140121623 A, Biggs
- US 4041203 A, Brock
- US 6075179 A, McCormack
- US 6111163 A, McCormack s
- US 2015099086 A, Cho
- US 4699823 A, Kellenberger
- US 5458592 A, Abuto
- US 5601542 A, Melius
- US 6129720 A, Blenke
- US 7938813 B, Elliker

- US 20020183703 A, Dodge
- US 5364382 A, Latimer
- US 6437214 B, Bolwerk
- US 6534149 B, Mace
- US 6617490 B, Lindsay